Europäisches Patentamt

European Patent Office

Office européen des brevets

(11) Veröffentlichungsnummer: **0 168 745**
**A2**

# (12) EUROPÄISCHE PATENTANMELDUNG

(21) Anmeldenummer: **85108436.8**

(22) Anmeldetag: **08.07.85**

(51) Int. Cl.⁴: **C 12 N 5/00**, C 12 P 21/00,
C 12 N 15/00, C 07 K 3/18,
C 07 K 17/00, C 12 P 21/02,
G 01 N 33/577
// C07K15/26 ,(C12P21/00,
C12R1:91)

(30) Priorität: **14.07.84 DE 3426077**

(71) Anmelder: **Dr. Karl Thomae GmbH, Postfach 1755,
D-7950 Biberach (Riss) (DE)**

(43) Veröffentlichungstag der Anmeldung: **22.01.86
Patentblatt 86/4**

(72) Erfinder: **Berthold, Wolfgang, Dr., Amriswilstrasse 7,
D-7950 Biberach/Riss (DE)**
Erfinder: **Zahn, Gabriele, Dr. Dipl.-Biol., Schlehenhang 4,
D-7950 Biberach/Riss (DE)**

(84) Benannte Vertragsstaaten: **AT BE CH DE FR GB IT LI LU
NL SE**

(54) **Monoklonale Antikörper gegen humanes IFN-gamma, diese Antikörper produzierende Hybridzellinien, Verwendung der neuen Antikörper und Verfahren zu deren Herstellung.**

(57)    Die Erfindung betrifft eine neue Immunglobulin-produzierende Hybridzellinie, welche durch Zellfusion von Myelomzellen mit gegen IFN-γ immunisierten Mäuse-Milzzellen nach bekannten Methoden hergestellt wird, sowie die von diesen Zellen gebildeten Antikörper und deren Verwendung bei der Reinigung und dem Nachweis von natürlichem oder rekombinantem IFN-γ und deren Unterscheidung.

EP 0 168 745 A2

ACTORUM AG

DR. KARL THOMAE GMBH　　　　　　　　　Case 12/014

D-7950 Biberach 1　　　　　　　　　　　Dr.So/Upp/Kp

Monoklonale Antikörper gegen humanes IFN-γ, diese Antikörper produzierende Hybridzellinien, Verwendung der neuen Antikörper und Verfahren zu deren Herstellung

---

Interferone wurden auf Grund ihrer besonderen biologischen Eigenschaften, nämlich durch ihre schützende Wirkung auf Zellen gegen Infektion durch Viren, entdeckt (1).

Relativ früh wurde erkannt, daß es verschiedene Arten von Interferonen gibt, die biologische Wirkungen zeigen. Antisera konnten mindestens drei Arten unterscheiden: IFN aus Leukozyten (IFN-$\alpha$), IFN aus Fibroblasten (IFN-ß) und Immuninterferon (IFN-γ). IFN-γ wird nur von kompententen Immunzellen ausgeschüttet, die durch spezifische Antigene oder Mitogene stimuliert werden.

Der deutlichste Beweis, daß das IFN-γ keine Identität oder Verwandtschaft mit den $\alpha$- und ß-Interferonen hat, ergab sich aus dem Vergleich der Aminosäuresequenzen, die man aus der Nucleotid-Sequenz klonierter Interferone ableiten konnte (2 und 3).

Durch seine biologischen Eigenschaften scheint sich insbesondere IFN-γ als interessantes und vielversprechendes Mittel bei der Behandlung viraler Erkrankungen einsetzen zu lassen. Eingehendere Untersuchungen scheiterten zunächst an der äußerst geringen Konzentration an IFN-γ in natürlichen humanen Zellen. Erst durch die Entwicklung der Gentechnologie war es nach langwierigen und schwierigen Untersuchungen möglich, auch IFN-γ in Mikroorganismen zu exprimieren. Dieses rekombinante IFN-γ (rIFN-γ) zeigte in hochgereinigter Form die gleichen Wirkungen wie das natürliche Interferon-γ. Es ist in beliebigen Mengen herstellbar und daher zum Einsatz in der Therapie viraler Erkrankungen geeignet.

IFN-γ trägt an mehreren Stellen verschiedene Zuckerreste. Die Art dieser Glykolisierung hängt von dem Organismus ab, in dem das Protein zur Expression gelangt. In manchen Organismen, beispielsweise in Escherichia coli, wird das Protein in nichtglykosilierter Form exprimiert. Für die biologische Aktivität ist die Glykosilierung jedoch ohne Bedeutung.

Wissenschaftliche Untersuchungen ebenso wie die therapeutische Anwendung ließen das Bedürfnis entstehen, nicht nur die Aktivität des Interferons, sondern das Protein selber nachzuweisen. Die biologische Aktivität auf lebende Zellen ist nur mühsam und aufwendig nachzuweisen.

Alternative Testsysteme müssen jedoch eine ausreichende Spezifität aufweisen, um IFN in biologischen Flüssigkeiten, die viele verschiedene Proteine enthalten, nachweisen zu können. Diese Forderung ist nur durch immunbiologische Methoden zu erfüllen. Eine Möglichkeit zum Aufbau eines immunbiologischen Testsystems besteht in der Verwendung von Antikörpern, die aus dem Serum von Tieren gewonnen werden, die mit dem nachzuweisenden Antigen immunisiert wurden. Seit den Arbeiten von Köhler und Milstein (15) läßt sich die Spezifiät solcher Antikörper-Testsysteme durch die Verwendung sogenannter monoklonaler Antikörper erhöhen.

Der vorliegenden Erfindung lag die Aufgabe zugrunde, neue monoklonale Antikörper zu entwickeln, die zum immunologischen Nachweis von IFN-γ geeignet sind.

Beim Einsatz des rekombinanten IFN-γ zu Therapiezwecken ist es bedeutsam, den Interferonspiegel überwachen zu können. Dabei kann es entscheidend sein festzustellen, ob das nachweisbare Interferon körpereigen oder exogener Natur ist. Die einzigen Unterschiede zwischen natürlichem und in E. coli exprimiertem IFN-γ besteht, wie bereits oben angeführt, in der unterschiedlichen, nicht für die biologische Aktivität relevanten Glykosilierung des Proteins.

Eine weitere Aufgabe der Erfindung lag daher in der Entwicklung neuer monoklonaler Antikörper, die durch ihre unterschiedliche Spezifität zwischen natürlichem und rekombinantem Interferon differenzieren können.

Untersuchungen des rIFN-γ ergaben, daß es im Vergleich zum natürlichen IFN-γ am N-terminalen Ende drei zusätzliche Aminosäuren trägt. Nach der für die Sezernierung des Proteins erforderlichen Leadersequenz von 20 Aminosäuren folgen drei Aminosäuren Cys-Tyr-Cys am rIFN-γ, die für das biologische Wirkungsspektrum ohne Bedeutung sind.

Es stellte sich jedoch heraus, daß ein um diese Aminosäuren verkürztes rIFN-γ erheblich stabiler ist und daher für die therapeutische Anwendung wertvoller sein kann. Aus diesem Grund wurde ein verkürztes rIFN-γ entwickelt (rIFN-γ[Cys-Tyr-Cys]).

Als weitere Aufgabe lag der vorliegenden Erfindung daher die Entwicklung neuer, monoklonaler Antikörper zugrunde, die mit dem natürlichen, dem rekombinanten und dem verkürzten rekombinanten IFN-γ unterschiedlich reagieren und dadurch eine Unterscheidung ermöglichen.

Gegenstand der vorliegenden Erfindung sind somit neue monoklonale Maus-Antikörper mit Spezifität für humanes natürliches und rekombinantes IFN-γ und deren Derivate gemäß den Patentansprüchen 6 bis 9. Die gewonnenen monoklonalen Antikörper sind für den immunologischen Nachweis dieser Peptide und deren Reinigung in immunoaffinitäts-chromatographischen Verfahren sehr gut geeignet. Ferner sind die Antikörper-produzierenden Hybridzellinien gemäß den Ansprüchen 1 bis 5 und Verfahren zu ihrer Herstellung Gegenstand der Erfindung.

Die Herstellung der monoklonalen Antikörper erfolgte in Anlehnung an bekannte Methoden (4):

Durch wiederholte Injektion einer geringen Menge des gereinigten, rekombinanten IFN-γ's aus E.coli wurden Balb/c Mäuse immunisiert. Sobald im Serum genügend Antikörper nachweisbar waren, wurden die Milzzellen dieser Tiere mit Myelomzellen fusioniert und die Hybride kultiviert.

Die einzelnen Kulturen wurden auf ihren Gehalt an spezifischen Antikörpern gegen rIFN-γ mit Hilfe eines Screeningtestes untersucht. Nach der Methode des "limiting dilution cloning" wurden Kolonien isoliert, die sich von Einzelzellen ableiteten. Auf diese Weise wurden drei Hybridzellinien gewonnen, die sich durch die Sekretion monoklonaler Antikörper mit unterschiedlichen Eigenschaften auszeichneten, nämlich die Hybridzellinien GZ-4, GZ-25 und C-15.

Zur Vermehrung wurden die Hybridzellen sowohl in vitro als auch in vivo kultiviert. Die hohe Vermehrungsrate in vivo machte dieses Kulturverfahren besonders günstig. Dazu wurden Zellen der einzelnen Hybridstämme in mit Pristan vorbehandelte Balb/c-Mäuse intraperitoneal injiziert. Der dadurch entstandene Ascitestumor wurde nach ca. 14-18 Tagen abgeerntet.

Die Isolierung der monoklonalen Antikörper gegen IFN-γ erfolgte durch die Aufreinigung der in vitro Zellkulturüberständen oder der Ascitesflüssigkeit. Die Reinigung wurde mittels einer geeigneten Anionenaustauschersäule, beispielsweise einer DEAE-Ionenaustauschersäule von Pharmacia durchgeführt.

Die Charakterisierung der molekularen Eigenschaften der Antikörper ergab:

- Das Molekulargewicht der gereinigten Antikörper beträgt > 150.000 Dalton (Bestimmung durch Polyacrylamidgelelektrophorese).
- Die spezifische Aktivität der Antikörper kann durch Kaninchen Anti Maus Immunglobulin Antiserum präzipitiert werden.
- Alle drei Antikörper (GZ-25, GZ-4, C-15) sind vom Typ IgG1, wobei die schwere Kette γ1, die leichte Kette $\chi$ ist (Bestimmung durch subtypenspezifische Antikörper in einem ELISA).

Molekulare Eigenschaften der Antikörper:

| | |
|---|---|
| Molekulargewicht: | > 150.000 Dalton |
| Spezies: | Maus |
| Typ: | IgG 1 |
| Schwere Kette: | γ1 |
| Leichte Kette: | $\chi$ |

Sollen Antikörper zum Nachweis eines bestimmten Antigens dienen sind im Prinzip 2 Assayanordnungen möglich. In beiden muß, wenn es keinen natürlichen Marker im Antigen gibt, eine Markierung einer der Komponenten vorgenommen werden: Erfolgt diese am Antigen, z.B. durch ein radioaktives Marker-Isotop, wird meist ein kompetitiver Verdrängungsassay benutzt wie der bekannte Radioimmunoassay (RIA). Erfolgt die Markierung

am Antikörper, wird als bevorzugter Assaytyp ein Immuno-Radiometrischer Assay (IRMA) oder ein Enzyme Linked Immunosorbant Assay (ELISA) eingesetzt. Es gibt sehr viele Möglichkeiten der Markierung. Als Beispiele sind Radiomarkierung, Fluoreszenzmarkierung, Kopplung von Markerenzymen, Kopplung von Metallchelaten zu nennen.

Eine andere Möglichkeit, die auch zu einer höheren Empfindlichkeit des Nachweises führen kann, besteht in der Kopplung der Antikörper mit niedermolekularen Haptenen. Diese können dann ihrerseits durch eine zweite Reaktion spezifisch nachgewiesen werden. Gebräuchlich sind z.B. Biotin (reagierend mit Avidin) oder Dinitrophenyl, Pyridoxal, Fluoreszamin (reagierend mit jeweils spezifischen Antihapten-Antikörper).

Die besonderen Bedingungen eines Antigens, und des Anwendungszwecks, geben für die Art des Assay und der Markierung Auswahlkriterien vor. In dem vorliegenden Fall wird für das Antigen + IFNγ ein leicht durchführbarer, schneller und empfindlicher Assay gefordert.

Die exquisite Spezifität von monoklonalen Antikörpern erlaubt es den empfindlicheren Assaytyp, d.h. die Markierung des Antikörpers zu wählen. Als Markierungssignal eignen sich besonders Enzyme, da die Anwendung von Radioisotopen mit großem Aufwand zum Schutz der Ausführenden und der Umwelt verbunden ist. Außerdem stellt die katalytische Aktivität eines Enzyms zudem eine Amplifikation des Signals dar. Besonders geeignet ist die Meerrettichperoxidase, da es eine Reihe von sehr guten Substraten gibt. Zudem kann das kleine Enzym mit einer einfachen, aber spezifischen Methode (Perjodatmethode) an Antikörper gebunden werden.

Alle erfindungsgemäßen gereinigten Antikörper wurden auf 3 Arten markiert: direkte Radiomarkierung mit $^{125}$J durch Jodierung der Tyrosinreste (8), indirekte Radiomarkierung nach der Methode von Bolton und Hunter (8), kovalente Bindung mit Meerrettichperoxidase nach der Periodatmethode (10). Die Radiomarkierungen wurden lediglich für Untersuchungen der Verschiedenheit der Antikörper-Bindungsstellen verwendet.

Die relative Lage der Bindungsstellen für die einzelnen Antikörper auf dem rIFN-γ Molekül wurde durch kompetitive Bindung der Antikörper untersucht.

Mikrotiterplatten wurden mit den drei verschiedenen Antikörpern (nach einer üblichen Methode (11)) beschichtet, und mit einer identischen Menge von rekombinantem rIFN-γ beladen. Nicht gebundenes rIFN-γ wird weggewaschen. Ein zweiter markierter Antikörper wird mit dem immobilisierten Komplex inkubiert. Es wurde untersucht, ob in dieser Konstellation Antikörper für ähnliche Bindungsstellen auf dem IFN-γ-Molekül rivalisieren. Dabei zeigte sich, daß selbst ein und derselbe Antikörper in der Beschichtungsphase γ-IFN immobilisieren und zusätzlich in markierter Form in der Nachweisphase an das Molekül binden kann. Daraus ist zu schließen, daß identische Bindungsstellen mindestens 2 mal pro Antigenmolekül vorhanden sind. Das Antigen rIFNγ liegt daher in einer polymeren Form, wahrscheinlich als Dimer, vor.

Der gleiche Befund zeigte sich auch bei der Verwendung von natürlichen IFN-γ's als Antigen.

Bei gleichzeitiger Zugabe markierter Antikörper und konkurrierender nicht markierter Antikörper zeigte sich in allen Kombinationsmöglichkeiten der 3 monoklonalen Antikörper eine kompetitive Hemmung. Es ist möglich, daß dieses Ergebnis durch eine sterische gegenseitige Hinderung der großen Antikörpermoneküle auf dem viel kleineren Proteintarget zu erklären ist, ohne daß damit die spezifischen Bindungsstellen für die Antikörper identisch oder nahe benachbart sein müssen.

Die Aktivität des rIFN-γ und eines internationalen NIH-Standards für humanes natürliches IFN-γ (Gg 23-901-530) wurden in einem üblichen antiviralen Assay bestimmt. Proben beider IFN-γ Präparate wurden mit einem Überschuß an Antikörpern von mindestens 1000-fach (Protein zu Protein) eine Stunde bei 37°C inkubiert. Die verbleibende antivirale Aktivität beider IFN-γ Präparate wurde nur durch den Antikörper GZ-4 neutralisiert (Tabelle 1).

Dieser Befund läßt sich durch die Annahme erklären, daß sich auf dem Antigen unterschiedliche Regionen befinden, die mit den verschiedenen Antikörpern unterschiedlich reagieren: nur der GZ-4 monoklonale Antikörper reagiert mit einer für die biologische Aktivität verantwortlichen Region.

Wie gezeigt wurde, liegen die aktiven γ-Interferone als Dimere vor. Da GZ-4 sowohl mit dem natürlichen als auch mit dem rekombinantnen IFN-γ reagiert, konnte ein Standardassay mit diesem monoklonalen Antikörper aufgebaut werden.

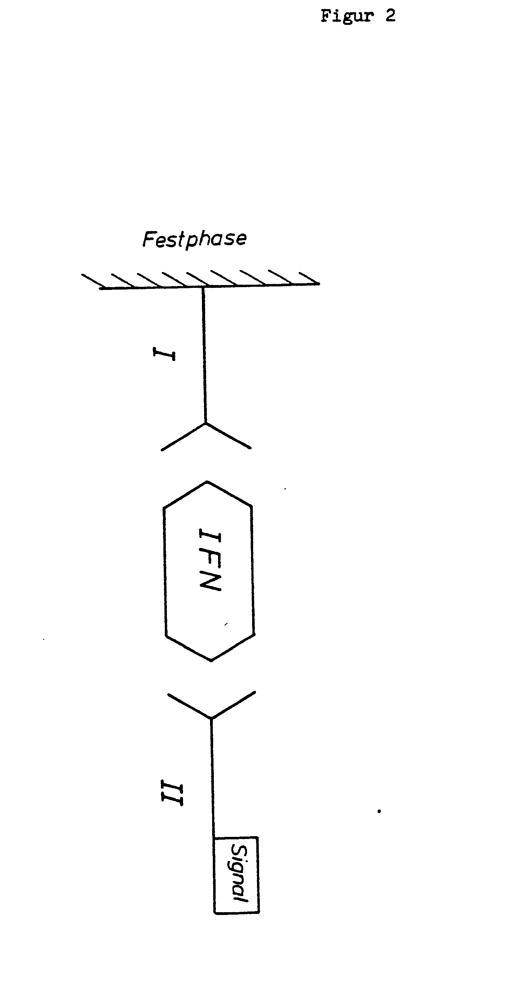

Der hier beschriebene Assay besteht aus GZ-4 in markierter und nicht markierter Form und stellt einen Festphasen-Sandwich ELISA dar (Figur 2). Ein nicht markierter Antikörper GZ-4 wurde an eine Oberfläche, z.B. Mikrotiterplatten, passiv adsorbiert oder kovalent gebunden, die Oberfläche in bekannter Weise gegen unspezifische Bindung blockiert und Interferon-haltige Proben wurden aufgelegt und inkubiert. Nachdem der Überschuß an IFN-haligen Proben wieder abgewaschen worden war, wurde ein markierter Antiköper GZ-4 (Radiomarkierung, Enzymmarkierung) mit dem immobilisierten IFN-γ inkubiert. Als Enzym eignet sich die Meerrettichperoxidase. Es konnte mit Konjugaten des Antikörpers GZ-4 gezeigt werden, daß IFN-γ mit einer Empfindlichkeit von 0,5 ng/ml in Proben von 0,1 ml nachzuweisen ist. Dies entspricht etwa 5-15 IE/ml. Die spezifische Aktivität des rekombinanten Interferons konnte mittels eines neuen NIH-Standards (Gg 23-901-530) aus natürlichem IFN-γ zu ca. $10^7$ IE/mg gemessen werden.

Wie bereits oben erwähnt, hatte die Analyse des N-terminalen Endes des natürlich vorkommenden IFN-γ ergeben, daß die ersten drei Aminosäuren nach der aus der Nucleotidsequenz ersichtlichen Leadersequenz von 20 Aminosäuren, nämlich $Cys^1-Tyr^2-Cys^3(-Gln^4)-$, nicht zu finden sind. Daher wurde eine neue Form des rIFN-γ entwickelt, die diese Aminosäuren nicht mehr enthält (12).

Mikrotiterplatten wurden mit beiden Formen des rIFN-γ beschichtet und die Bindung aller 3 Antikörper an diesen immobilisierten IFN-γ-Typen mit markierten Anti-Mausimmunoglobulin-Antikörpern nachgewiesen.

Alternativ konnte durch Beschichtung mit den verschiedenen Antikörpern die Bindung der verschiedenen IFN-γ-Moleküle durch ein GZ-4-Peroxidasekonjugat nachgewiesen werden (Tabelle 2). Der Antikörper C-15 bindet nur an rekombinantes IFN-γ, während er nicht an natürliches IFN-γ binden kann. Der Antikörper GZ-25 bindet mit hoher Affinität an das Interferon mit dem N-terminalen Ende Cys-Tyr-Cys und mit einer sehr viel geringeren Affinität an das rekombinante Interferon ohne diesen N-Terminus, aber nicht an natürliches IFN-γ. Der Antikörper GZ-4 kann alle getesteten IFN-γ-Typen binden.

Zusammenfassend läßt sich feststellen, daß

1. den drei Antikörpern, ein Molekulargewicht von $\geq$ 150 000 Dalton gemeinsam ist und die schwere Kette γ1, die leichte ϰ ist.

2. Die Ergebnisse beweisen die Unterschiedlichkeit der von den Antikörpern erkannten Regionen auf den IFN-γ Molekülen:

a) Der Antikörper GZ-4 weist, unabhängig vom Vorhandensein der N-terminalen Sequenz $Cys^1$-$Tyr^2$-$Cys^3$-, eine neutralisierende Wirkung auf die antivirale Aktivität von natürlichem und rekombinantem IFN-γ auf.

b) Der Antikörper C-15 zeigt keine neutralisierende Wirkung auf die antivirale Aktivität von rekombinantem IFN-γ, jedoch eine bindende Wirkung auf rIFN-γ, unabhängig vom Vorhandensein der Sequenz $Cys^1$-$Tyr^2$-$Cys^3$- , und überhaupt keine Wirkung auf NIH IFN-γ.

c) Der Antikörper GZ-25 übt keine neutralisierende Wirkung auf die antivirale Aktivität von rekombinantem IFN-γ aus, jedoch eine bindende Wirkung auf rIFN-γ, welches die Sequenz $Cys^1$-$Tyr^2$-$Cys^3$- enthält, während die Bindung auf rIFN-γ, das diese Sequenz nicht enthält, stark reduziert ist; er weist keine bindende Wirkung auf NIH IFN-γ auf.

3.) Auf Grund der unterschiedlichen bindenden Wirkung und/oder neutralisierenden Wirkung auf die antiviralen Eigenschaften von IFN-γ und seinen Derivaten der neuen monoklonalen Antikörpern werden erstmals Testmöglichkeiten gegeben, diese Arten von IFN-γ-Molekülen zu unterscheiden.

4.) Die biologisch aktive Form des IFN-γ ist ein Dimer aus identischen Monomeren. Daher ist ein Immunoassay mittels eines einzigen Antikörpers in markierter und nicht markierter Form, z.B. zur Immobilisierung und zum Nachweis durchführbar. Ein solcher Assay kann zur Unterscheidung von inaktiven Monomeren des IFN-γ und aktiven Dimeren benutzt werden. Ein besonders gut geeigneter Antikörper für den spezifischen Assay von allen IFNγ-Typen ist GZ-4. Ein empfindlicher Immunoassay des Sandwichtypes mit nur diesem einen Antikörper wird beschrieben.

Die nachfolgenden Beispiele sollen die Erfindung näher erläutern:

Beispiel 1

a) Immunisierung:

Etwa 4 Wochen alte, weibliche BALB/c Mäuse wurden mit je
0,4 ml einer Emulsion aus 100 µg rIFN-γ, 50 µg Muramyldipeptid (der Fa. Institut Pasteur Produktion, Paris) und 200 µl
Mineralöl bzw. inkomplettes Freund Adjuvans intraperitoneal
und/oder subkutan immunisiert. Nach 3 Wochen erfolgte eine
2. Immunisierung mit derselben Menge Antigen, jedoch ohne
Mineralöl intraperitoneal. 3 Tage später wurde die Milz von
1 oder 2 Tieren steril entnommen.

b) Fusion:

Eine Zellsuspension wird durch mechanische Zerkleinerung
(Verreiben durch ein Metallsieb) hergestellt, und die entstehende Suspension von Zellen wird 2 x mit isotoner Kochsalzlösung gewaschen und zentrifugiert. Die Gesamtmenge an
isolierten Milzzellen einer Milz betrug gewöhnlich
$10^8$ Zellen. Erfolgreiche Fusionen wurden mit $10^7$ Milzzellen durchgeführt. Der Rest der Milzzellen konnte in Proben von $10^7$ Zellen pro ml in 90 % fötalem Kälberserum,
10 % DMSO bei -196°C eingefroren werden. Die so gelagerten
immunkompetenten Zellen waren nach Auftauen zu 80 % lebensfähig und unterschieden sich in ihrer Eignung für weitere
Fusionen nicht von frisch isolierten Milzzellen. Die Myelomzellen wurden 2 x in serumfreiem Zellkulturmedium gewaschen
und bei 1000 x g zentrifugiert. Die beiden Zellpellets
(Myelomzellen und Milzzellen) wurden in serumfreiem Kulturmedium resuspendiert und zusammen bei 1000 x g zentrifugiert. Das Mengenverhältnis betrug 1 x $10^7$ Myelomzellen zu
1 x $10^7$ Milzzellen. Der Überstand wurde nach der Zentrifugation entfernt.

Die Fusion der Zellmischung wurde mit 45 % Polyäthylenglykol 6000, 5 % Dimethylsulfoxid in Wasser bei 37°C über 90 Sekunden durchgeführt. Die Suspension wurde langsam mit Medium verdünnt und die Zellen einmal gewaschen. Das Zellsediment wurde vorsichtig unter Rühren in Selektionsmedium suspendiert und auf Kulturnäpfchen verteilt. Die Zellen eines Fusionsansatzes wurden auf 100 bis 200 Näpfchen von 2 cm$^2$ Bodenfläche gegeben. Diese Näpfchen enthielten am Tag zuvor etwa 10 000 - 50 000 peritoneale mononucleäre Zellen, vornehmlich Makrophagen als Fütterzellen (feed cells).

Die Selektion durch HAT-Medium erfolgte nach Littlefield (13) und erlaubte nur Hybriden aus Myelomzellen (für permanentes Wachstum) und Milzzellen (mit funktioneller HPRT) ein Wachstum, alle anderen Zellen starben ab. Den Zellen wurde alle 2-3 Tage frisches Medium zugefügt. Als normales Wachstumsmedium wurde eine Lösung aus Dulbecco's Modified Essential Medium (DMEM) mit 15-20 % hitzeinaktiviertem fötalem Kälberserum, 100 µg/ml Natriumpyruvat, 100 µg/ml Gentamycin, 50 µM Mercaptoäthanol und frischem Glutamin, zu 2 mM zugesetzt. Der Zusatz von HAT wurde bis zu einer Endkonzentration von 100 µM Hypoxanthin, 40 nM Aminopterin und 160 nM Thymidin vorgenommen.

Nach 2-3 Wochen wurden die Überstände der Näpfchen auf ihren Gehalt an spezifischen Antikörpern getestet. Zellen aus den Näpfchen mit spezifischen Antikörpern wurden zweifach in Mikrotiterplatten durch Limit Verdünnung kloniert.

Zur Klonierung nach der Methode des "limiting dilution cloning" wurden die Zellen zu 1, 5, 10, 25 Zellen pro 200 µl Medium verdünnt und auf 48-96 Näpfchen einer Mikrotiterplatte mit 200 µl pro Napf verteilt. Als Fütterzellen enthielt jedes Näpfchen ca. 1 000 - 5 000 peritoneale Makrophagen. Zur Testung der Überstände wurde diejenige Zellverdünnung gewählt, bei der nach der Statistik Einzelklone auftreten konnten, d.h. nur 37 % aller Näpfchen durften Zellbewuchs zeigen.

Die Überstände von bewachsenen Näpfchen wurden zunächst auf den Gehalt von Maus-Antikörpern getestet. Nur positive Überstände wurden dann zur Testung auf ihre rIFN-γ Spezifität gebracht. Bei einzelnen Klonierungen waren die Anwuchsrate der Zellen, d.h. die Klonierungseffizienz der Zellen, sehr unterschiedlich. Es gab Klonierungen, wo selbst bei dem Einsatz von einer Zelle pro Napf 100 % Bewuchs erfolgte, und andere, z.B. bei 25 Zellen pro Napf Einsatz, wo nur wenige Näpfchen bewachsen waren. Gleichermaßen war die Antikörpersekretion der Klone unterschiedlich und schwankte von 0 % bis 100 % in einer nicht vorhersehbaren Art und Weise.

Von 200 angelegten Ur-Näpfchen aus 2 Fusionen zeigten 98 % Zellbewuchs. Von diesen zeigten ca. 11 positive Ergebnisse im rIFN-γ Screeningtest. Von diesen wurden 4 wiederholt positiv gewertet. Drei monoklonale Hybridome wurden weiterverfolgt: GZ-4, GZ-25 und C-15.

c) Myelomzellsubklonierung

Die Zellinie P3X63 Ag 8.653 die im Labor gehalten wurde und üblicherweise als Myelomzelle bei Fusionen verwendet wurde, mußte wegen ihrer schlechten Wachstumseigenschaften neu kloniert werden. Dies erfolgte in Limitverdünnung auf Mikrotiterplatten und Ascitestumorpassage.

Balb/c Maus peritoneale Makrophagen zu 1 000 - 5 000 Zellen pro Näpfchen von 0,3 cm$^2$ wurden als Futterzellen eingesät. Zu diesen Näpfchen wurde an folgenden Tag P3X63 Ag 8.653 Zellen in unterschiedlichen Verdünnungen von 0.3 - 25 Zellen pro Napf zugegeben. Ein besonders schnell wachsender Klon wurde weitervermehrt bis ca. 50 x 10$^6$ Zellen vorhanden waren. Diese wurden zu 10$^7$ Zellen pro Tier in den Peritonealraum von ca. 50 Tage alten Mäusen (Balb/c) injiziert. Die Mäuse waren wenigstens 14 Tage zuvor mit je 0,5 bis 1 ml Pristan (Tetramethylpentadekan der Firma Roth) intraperitoneal vorbehandelt worden. Von 5 Tieren entwickelten 3 sichtbare "Wasserbäuche". Die Ascitesflüssigkeit wurde steril entnommen und die Zellen inspiziert. Zellen aus dem Ascites mit der höchsten Zelldichte wurden in Kultur genommen und mehrere Wochen mit bis zu 20 µg/ml 8-Azaguanin im Medium behandelt. Danach wurden die Zellen ein zweites Mal im Medium ohne 8-Azaguanin wie oben beschrieben kloniert. Ein gut wachsender Klon wurde weiter in vitro vermehrt und nochmals als Aszitestumor propagiert. Zellen dieses 2. Aszites wurden isoliert, gewaschen und in Aliquots von $10^7$ Zellen als Stammbank eingefroren. Für jede Fusion wurde ein Aliquot 14 Tage vor der Fusion aufgetaut und die Zellen zunächst eine Woche in 8-Azaguanin-haltigem Medium vermehrt. In der 2. Woche wurden die Zellen in normalem Medium gehalten, aber im 2 Tage Rhythmus verdünnt, damit sie sich am Tage der Fusion in logarithmischem Wachstum befanden.

## d) Screeningassay für spezifische Antikörper

Das reine, aus einem Konzentrat stammende rIFN-γ wurde in PBS (phosphatgepufferter Saline bestehend aus 0,8 % NaCl, 0,02 M Natriumphosphat eingestellt mit HCl oder NaOH auf pH 7.4) auf 3 µg/ml verdünnt. Näpfchen von geeigneten Mikrotiterplatten, z.B. der Fa. Nunc (Roskilde, Dänemark), werden mit 100 µl dieser Lösung beschickt. Nach 1 bis 16 Stunden bei 4°C wurde der Überstand abgesaugt und die Näpfchen mit 200 µl einer 1,5 % BSA (Bovines Serumalbumin) PBS-Lösung mindestens eine Stunde behandelt. Danach wurde der Überstand verworfen. Je 100 µl Zellkulturmedium von Überständen der wachsenden Hybridomklone oder Serum-Verdünnungen von immunisierten Mäusen wurden für mindestens 2 Stunden (bis maximal 24 Stunden) bei Raumtemperatur (oder 4°C) inkubiert. Die Näpfe wurden mit je 200 µl PBS oder Wasser mehrfach gewaschen. Dann wird 100 µl einer geeigneten Konzentration von AntiMausimmunoglobulin-Antikörpern für 1 Stunde aufgelegt und bei Raumtemperatur inkubiert. Diese Antikörper waren mit einem Marker versehen, z.B. mit $^{125}J$, oder mit dem Enzym Meerrettichperoxidase gekoppelt. Näpfchen die positive Ergebnisse zeigten (Enzymreaktion bzw. Radioaktivität), enthielten antigenspezifische Antikörper.

Der Nachweis von Mausantikörpern wurde analog dem spezifischen Screeningassay durchgeführt, nur wurden die Mikrotiterplattennäpfchen - statt mit rIFN-γ mit einem Anti-Maus-immunoglobulin Antikörper, z.B. von Kaninchen, beschichtet.

e) Antikörperherstellung:

Monoklonale Hybridome wurden in Kultur vermehrt. Je $10^7$ Zellen in PBS wurden in den Peritonealraum von mit Pristan vorbehandelten jungen weiblichen Balb/c Mäusen appliziert (siehe Beispiel 1c). Ascitesbildung erfolgte zunächst bei 2 bis 4 von 5 Tieren. Aus diesen Tumoren entnommene Asciteszellen wurden dann direkt zu ca. $10^7$ Zellen pro Maus weiter passagiert und ergaben 100 % Anwachsraten.
Nach dem Ernten der Tumore wurde die Ascitesflüssigkeit zunächst durch Zentrifugation (2000 rpm 10 Minuten) von Zellen befreit und gepoolt. Der zellfreie Pool wurde bei 100 000 x g 45 Minuten bei 4°C zentrifugiert, um unlösliche Präzipitate zu sedimentieren und Lipide durch Flotation zu entfernen. Die klare Lösung zwischen Lipidpfropfen und Sediment wurde abgepumpt und mit einem Volumen einer bei 4°C gesättigten Ammonsulfatlösung versetzt. Nach mindestens 2 Stunden bei 4°C wurde das Präzipitat sedimentiert (12 000 x g, 30 Minuten bei 4°C) und in einer TBS Lösung (Tris(hydroxymethyl)aminomethan 0,02M, 0,14M NaCl mit HCl auf pH 8,5 eingestellt) zu einer Proteinkonzentration von 10-20 mg/ml wieder gelöst.

Die Lösung wurde gegen einen 100-fachen Überschuß an TBS für 2 Tage dialysiert, wobei der Dialysepuffer mehrfach gewechselt wurde. Unlösliches Material wurde sedimentiert (bei 12 000 x g, 30 Minuten bei 4°C). Der geklärte opaque Überstand wurde über eine Ionenaustauschsäule des Types DEAE (Diethylaminoethyl) wie z.B. DE 52 Cellulose (Whatman) oder DEAE-Sepharose 4B (Pharmacia) gebunden. Die nicht adsorbierte Fraktion wurde aufgefangen und konnte weiter durch eine Proteinfällung bei einer Konzentration von 14 % mittels Polyethylenglykol 6000 gereinigt werden. In dieser Form ist der gereinigte Antikörper gut geeignet für Lagerung und Versand.

Das weiße Präzipitat wurde durch Zentrifugation gesammelt und konnte wieder in einem gewünschten Lösungsmittel gelöst werden, z.B. in PBS.

Zellkulturüberstände von Hybridenkulturen, die aber meist nur eine sehr viel niedrigere Antikörperkonzentrationen besaßen - bis maximal 100 µg/ml verglichen mit bis zu 20 mg/ml in Ascites - konnten ebenso behandelt werden. Die Ultrazentrifugation war dabei allerdings nicht nötig, da diese Medien kaum Lipide enthielten.

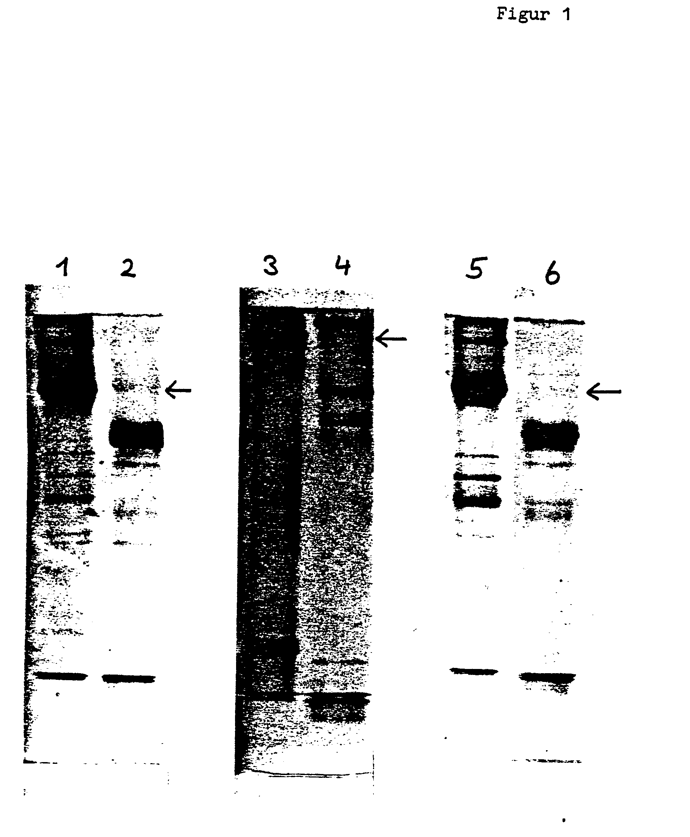

Die Reinigung der verschiedenen Antikörper wurde mittels der SDS-Polyacrylamidgelelektrophorese durchgeführt und nach Laemmli (7) getestet. Ein typisches Beispiel wird in Fig. 1 dargestellt.

Beispiel 2

Markierung der neuen Antikörper

a) Direkte Iodierung (8)

Gereinigte Antikörper wurden in einer Konzentration von 0,5-1,0 mg/ml in PBS pH 7,5 gelöst. Eine Probe von 5-10 µg in dem entsprechenden Volumen wurde zu einer Lösung von 0,5-1 mCi $^{125}$I in 100 µl 0,5 M Natriumphosphat pH 7,5 gegeben, 30 µl Chloramin T, frisch gelöst zu 0,4 mg/ml in 0,5 M Natriumphosphat pH 7,5, wurde zugegeben und sofort vermischt. Nach 30 bis 90 Sekunden wurde 100 µl Natriummetabisulfit, gelöst zu 2 mg/ml in 0,5 M Natriumphosphatlösung, zugegeben. Die Gesamtprobe wurde nach wenigen Minuten durch Gelpermeation in einer Chromatographiesäule von ca. 10 ml Bettvolumen, gefüllt mit Sephadex G 10 oder G-25 (Pharmacia) in einem Puffer (z.B. 0,5 % BSA in PBS) aufgetrennt. Die radioaktivmarkierten der Antikörper wurden in den ersten Fraktionen, die aus der Säule eluiert wurden, aufgefangen. Spezifische Aktivitäten von 1-10 µCi $^{125}$I pro µg Protein wurden routinemäßig gefunden, bei Ausbeuten von bis zu 20 % der Radioaktivität im Protein.

Analog, aber zum Teil einfacher durchführbar waren Radiomarkierungen mittels immobilisierter Lactoperoxidase und Glucoseoxidase. Bevorzugt wurde die Iodogen $^R$ Methode (14) verwendet:

Die oxidierende Substanz 1,3,4,6-Tetrachlor-3a,6a-diphenylglycoluril (als "Iodogen" von Pierce Chemicals erhältlich) ist nicht wasserlöslich. Sie wurde zunächst bei 40 µg/ml in Dichlormethan gelöst und die innere Oberfläche des Iodierungsgefäßes mit einer Menge von 50 µl, d.h. 2 µg Substanz, durch Verdampfen des Lösungsmittels beschichtet. Das Protein in 10 µl 0,5 M Natriumphosphat pH 7,0 wurde zu 20 µl 0,5 M Natriumphosphat pH 7,0 in das Gefäß gegeben und 10 ml Na $^{125}$J (etwa 0,5-1mCi) dazugemischt. Nach 10-30 Minuten bei Raumtemperatur wurde 210 µl Phosphatpuffer zugesetzt und die gesamte Probe wie oben beschrieben auf eine Sephadex G-10 Säule gegeben.

**b) Indirekte Iodierung nach (9)**

Das jodierte Tyrosinanaloge 3-(4-Hydroxyphenyl)-propionsäure als Hydroxysuccinimidester (= "Bolton-Hunter Reagenz") wurde von Amersham oder New England Nuclear bezogen. Die Probe mußte 0,5-1,0 mg/ml Protein enthalten und wurde vor der Iodierung excessive gegen 0,1 M Borat oder Phosphatpuffer pH 9,0 dialysiert, um Aminogruppen tragende niedermolekulare Substanzen zu entfernen. Durch Verdampfen des Lösungsmittels Benzol wurde die Gefäßwand mit dem Bolton-Hunter Reagenz beschichtet. Eine Menge von 0,5-1 mCi $^{125}$I als Na$^{125}$I wurde für 5-10 µg Protein eingesetzt. Die Proteinlösung wird in das trockene Reaktionsgefäß gegeben. Nach 15-30 Minuten bei 0°C wurde das überschüssige Reagenz durch Zugabe von 0,3 M Glycin in Boratpuffer pH 8,5 zerstört, das gesamte Reaktionsvolumen nach weiteren 30 Minuten auf eine Sephadex G-25 Chromatographiesäule, die in 0,5 % BSA oder 0,25 % Gelatine in 0,05 M Natriumphosphatpuffer pH 7,2 äquilibriert worden war, aufgebracht und gereinigt.

**c) Kopplung der Meerrettich Peroxidase nach (10)**

12 mg Meerrettichperoxidase (Boehringer Mannheim) mit der Reinheitszahl von ca. 3,0 wurde in 6 ml Wasser gelöst. 0,6 ml einer frisch hergestellten 0,1 M Natriumperjodatlösung (Merck, Darmstadt) wurde zugegeben und bei Raumtemperatur über 20 Minuten gerührt. Die Oxidation wurde durch Zugabe von einigen Tropfen Glycerin gestoppt und die Meerrettichperoxidase von dem Perjodat durch eine kurze Entsalzungschromatographie (Sephadex G 10 mit Bettvolumen von 20 ml, äquilibriert mit 1 mM Essigsäure pH 4,4) getrennt.

Durch Zugabe von 60 µl 0,2 M Natriumcarbonatpuffer pH 9,5 wurde ein alkalischer pH eingestellt und unmittelbar 24 mg Antikörper, gelöst in 2 bis 3 ml 0,01 M Natriumcarbonatpuffer pH 9,5, zugegeben. Die Kopplung erfolgte über 2 Stunden bei Raumtemperatur. Die überschüssigen Aldehydgruppen wurden durch Zugabe von 0,3 ml frisch bereiteter Natriumborhydrid-Lösung (4 mg/ml Wasser) in 2 Stunden bei 4°C reduziert. Das Konjugat und der eventuell noch freie Antikörper wurde durch Zugabe eines gleichen Volumens an Ammonsulfatlösung, die bei 4°C gesättigt worden war, gefällt. Nach mindestens 2 Stunden bei 4°C wurde das Präzipitat sedimentiert (10 000 x g, 20 Minuten, 4°C) und in 12 ml PBS gelöst. Das Konjugat konnte weitergereinigt werden oder wurde aliquotiert und benutzt.

Konjugate nach dieser Herstellung zeigten routinemäßig gute Eigenschaften für den beschriebenen ELISA in Endverdünnungen von 1 zu 30 000, d.h. ca. 0,4 µg/ml bezogen auf den Antikörpergehalt des Konjugats.

## Beispiel 3

### a) Aufbau eines Immunoassay:

Der Aufbau des spezifischen Assay ist schematisch dargestellt (Fig. 2).

Ein Antikörper I wird durch passive Adsorption oder kovalente Bindung an einen Träger (Kügelchen, Filter, Mikrotiterplatte aus Polystyrol oder Polyvinylchlorid, Glas, Filterpapier, Polyacrylamidgel oder anderen Materialien) immobilisiert. Die exquisite Spezifizität von monoklonalen Antikörpern erlaubt nur dem spezifischen Antigen, hier dem IFN-γ, durch Immunbindung über eine einzige molekulare Bindungsstelle des Antigens gebunden zu werden. Die Menge des bindbaren Antigens ist der Konzentration und Menge des Antigens in der Lösung proportional. Das Antigen wird durch Immunbindung des Antikörpers II an eine andere molekulare Bindungsstelle erkannt. Der Antikörper II trägt ein Signal. Die Menge des immobilisierbaren Signals ist damit direkt proportional der Menge des immobilisierten Antigens und damit auch der Konzentration des Antigens in der zu untersuchenden Lösung.

b) Durchführung des Assays:

1. Beschichtung:

a) Anhaftpuffer: Natrium-Bicarbonatpuffer pH 9,5
(NaHCO$_3$  8.4 g/l = 0,1 M)

b) Antikörper: eine Lösung von GZ 25 oder C 15 oder GZ 4 als ganzer Antikörper als Fab bzw. F(ab)$_2$ Fragmente in Konzentration 0,5 ml mit 0,2 mg/ml Antikörper wird 1:100 in 50 ml Anhaftpuffer auf 2-25 µg/ml verdünnt.

2. Verdünnungsmedium für IFN-Proben, Antikörper-Konjugat:

a) PBS-Puffer (Phosphatgepufferte Saline) pH 7,4:

1. 0,15 M NaCl                    (8,77 g/l)
   · 0,02 M Na$_2$HPO$_4$ x 2 H$_2$O    (3,56 g/l) sauer

2. 0,15 M NaCl                    (8,77 g/l)
   0,02 M NaH$_2$PO$_4$ x 2 H$_2$O      (3,12 g/l) alkalisch

Durch Mischen der beiden Lösungen wurde der Puffer auf pH 7,4 eingestellt.

b) PBS/BSA-Puffer (Bovines Serum Albumin):
   Zu PBS wurden 0,5 % BSA (5,0 g/l Serum-Albumin vom Rind, reinst, lyophilisiert, Behringwerke AG) gegeben.

3. Waschmedien:

a) Aqua dest.

b) Waschpuffer:
Zu 1 l PBS pH 7,4 wurden 0,15 % Tween 20 (1,5 g/l) gegeben.

4. Antikörper-Konjugat:

GZ-4-HRPO (Meerrettichperoxidase = Horse radish peroxidase) (das Konjugat wurde nach der Vorschrift im Beispiel 3 hergestellt).
Konzentration: Portionen mit 0,2 ml (1 :200 verdünnt mit PBS/BSA-Puffer) entsprachen einer Konzentration von ⪕ 100 ng/ml.
Gebrauchsverdünnung: 0,2 ml in 30 ml (1 :150) in PBS/BSA-Puffer (für eine Endverdünnung 1 : 30.000 im Näpfchen).

5. IFN-γ:

a) Natürliches IFN-γ (z.B. NIH Standard Gg 23-901-530)
b) rIFN-γ mit Cys-Tyr-Cys-N-Terminus
c) rIFN-γ ohne Cys-Tyr-Cys-N-Terminus
   filtriertes Konzentrat wurde jeweils mit PBS/BSA-Puffer zu einer Endkonzentration von 11 ng/ml Protein, bzw. 200 IE/ml verdünnt
   Testkonzentration 1:1 bis 1:16.

6. Substratlösungen:

a) Citronensäure-Puffer pH 5,0:
21 g Citronensäure/l + ca. 60 Plätzchen KOH
(Zugabe der Plätzchen bis zu einem pH von 5,0)

b) OPD (Ortho-Phenylendiamin):

173 mg OPD in 20 ml Citronensäure-Puffer, Lagerung bei +4°C.

c) $NaBO_2xH_2O_2x3H_2O$ (Natriumperborat rein = E. Merck, Darmstadt):

d) gleiche Anteile <u>Aqua dest.</u> = 20 ml:

Die Lösungen b, c und d wurden auf dem Magnetrührer gemischt. Die Substratlösung wurde erst kurz vor Gebrauch hergestellt, pro Vertiefung wurden 100 µl gegeben; die Reaktion sollte im Dunkeln erfolgen.

## 7. Reaktionsstop

4 N HCL 456 ml 32%iger HCl wurden mit Aqua dest. auf 1.000 ml ver-dünnt.

ELISA Nachweis von IFN-γ (GIFA)

| Arbeitsgang | Vol (µl) | Konzentration | Medium | pH | Temperatur °C | Zeit |
|---|---|---|---|---|---|---|
| 1. Beschichtung | 100 | 2 µg/ml C 15 oder 25µg/ml GZ-4 | $NaHCO_3$ | 9,5 | 4 | 16 Stunden |
| 2. Waschen 2mal | 200 | - | A. dest. | - | 16 - 22 | 10-30 Minuten |
| 3. Blockierung | 200 | 1,5 % BSA | Waschpuffer | 7,4 | 16 - 22 | 1-2 Stunden |
| 4. Waschen 2mal | 200 | - | A. dest. | - | 16 - 22 | 10-30 Minuten |
| 5. Verdünnung | 100 | 0,5 % BSA | PBS | 7,4 | 16 - 22 | 10-30 Minuten |
| 6. Gamma-IFN-Proben | 100 | 0,5 % BSA | PBS | 7,4 | 16 - 22 | 3 Stunden |
| 7. Waschen 3mal | 200 | | Waschpuffer | 7,4 | 16 - 22 | 10-30 Minuten |
| 8. Inkubation | 100 | GZ-4-HRPO | PBS/0,5 % BSA | 7,4 | 16 - 22 | 2 Stunden |
| 9. Waschen 6mal | 200 | - | Waschpuffer | 7,4 | 16 - 22 | 10-30 Minuten |
| 10. Substrat | 100 | OPD + $NaBO_2 \cdot H_2O_2 \cdot 3H_2O$ Citrat | | 5,0 | 16 - 22 | 30 Minuten (dunkel) |
| 11. Reaktionsstop | 100 | 4 N | HCl | 1,0 | 16 - 22 | bis 1 Stunde |

12. Ablesung: 200 µl in einer Mikrotiterplatte bei einer Wellenlänge von 492 nm innerhalb einer Stunde nach Zugabe von Stopreagenz.

Beispiel 4:

Neutralisation der antiviralen Aktivität von IFN-γ

Konfluente Kulturen von primären humanen Fibroblasten in
Mikrotiterplatten (Stamm: Phil33, isoliert von einer normalen Vorhaut) wurden mit IFN-γ in serologischen Verdünnungen inkubiert. Zur Kontrolle des Assay wurden Labor-
Standardlösungen von IFN-α und IFN-ß neben rIFN-γ aufgelegt.
Nach 16-24 Stunden wurden die überstehenden IFN-Lösungen
entfernt und die Zellen mit Virus VSV (Vesicular Stomatitis
Virus) infiziert. Nach einer Inkubation von 24-48 Stunden
werden die Zellen fixiert und gefärbt.

Der schützende Effekt des IFN zeigte sich durch die Färbbarkeit von intakten Zellen. Der zytopathische Effekt des Virus
ließe die Zellen lysieren und damit während der Färbung abschwemmen.

18 ng rekombinantes IFN-γ wurde mit je 20 µg der Antikörper
GZ-25, GZ-4 oder C 15 in 200 µl 1 Stunde bei 37°C inkubiert
und anschließend serologisch, in 1 zu 2 Schritten, in Kulturmedium verdünnt und auf seine antivirale Aktivität wie
oben beschrieben getestet.

In gleicher Weise wurde eine Probe des internationalen NIH-
Standard für humanes natürliches IFN-γ (Gg 23-901-530) mit
200 IE getestet. Alle Proben wurden in einem Ansatz getestet. Der Assay zeigte für IFN-ß und IFN-α eine Empfindlichkeit von 1 IE (bezogen auf IFN-α bzw. IFN-ß).

Der unbehandelte NIH IFN-γ Standard zeigte einen Endpunkt
bei 640, d.h. der Faktor 6,25 ist nötig um in diesem Assay
Internationale IFN γ-Einheiten abzulesen.

Rekombinantes IFN-γ hatte einen Endpunkt bei Verdünnung
1/254; daher eine spezifische Aktivität von ca.
17 x $10^6$ IE/mg Protein/ml. Die Neutralisation der antiviralen Aktivität ist in der Tabelle 1 mit + bezeichnet.

Tabelle 1:

Neutralisierung der antiviralen Aktivität von rIFN-γ:

| Antikörper | rek. IFN-γ | NIH IFN-γ |
|---|---|---|
| GZ-25 | - | - |
| C-15 | - | - |
| GZ-4 | + | + |

Beispiel 5:

Bindung der Antikörper am IFN-γ

Die Tabelle 2 stellt eine Zusammenfassung aus verschiedenen
Experimenten dar.

a) Mikrotiterplatten wurden mit den beiden oben beschriebenen Arten von rIFN-γ direkt beschichtet und mit den drei
Antikörpern inkubiert. Die Bindung der Antikörper wurde
durch Bindung von markiertem Kaninchen AntimausIg nachgewiesen.

b) Mikrotiterplatten wurden mit den drei verschiedenen Antikörpern beschichtet. Äquivalente Mengen von drei verschiedenen IFN-γ Präparaten wurden auf jede der Platten aufgebracht. Das immobilisierte IFN-γ wurde mittels des GZ-4-Per-oxidasekonjugats nachgewiesen.

In der Tabelle bedeuten:

+: IFN-γ wurde nachgewiesen

±: IFN-γ Nachweis war stark reduziert gegenüber der positiven Kontrolle auf der GZ 4 Beschichtung.

-: IFN-γ konnte nicht nachgewiesen werden.

Tabelle 2:

Bindung von verschiedenen IFN-γ-Formen:

Zusammenfasssung der Ergebnisse:

| Antikörper | r IFN-γ +(CysTyrCys) | r IFN-γ -(CysTyrCys) | NIH IFN-γ |
|---|---|---|---|
| GZ-25 | + | ± | - |
| C-15 | + | + | - |
| GZ-4 | + | + | + |

Referenzen:

(1) Übersicht: W.E. Stewart II, The Interferon System Springer Verlag Wien, New York, 1979

(2) Gray. P.W., Leung D.W., Pennica D. Melvaton E., Najarian R., Simonsen C.C., Derynck R., Sherwood L.J., Wallace D.M., Berger S.L., Levinson A.O., Goeddel D.V. in Nature 295, 503-508 (1982).

(3) Taniguchi T. Mantei N., Schwarzstein M., Nagata S., Muramatsu M., Weissmann C.in Nature 285, 547-549 (1980).

(4) Galfre G., Howe S.C., Milstein C., Butcher G.W., Howard J.C. in Nature 266, 550-552 (1977).

(5) Kearney J.F., Radbruch A., Liesegang B., Rajewsky K., J. Immuno 123, 1548-1550 (1979)

(6) Galfre G, Milstein C., in Methods in Enzymology Vol. 73,1-45. Langone J.J., van Vunalkis H. (Eds.) Academic Press (1981)

(7) Laemmli U.K. Favre M. in J. Mol. Biol. 80, 575-599 (1973).

(8) Greenwood F.C., Hunter W.H., Glover J.S. in Biochem. J. 89, 114-123 (1963).

(9) Bolton A.E., Hunter W.H. in Biochem. J. 133, 529-538 (1973).

(10) Wilson M.B., Nakane P.K. in "Immunofluorescence and related staining techniques"
Knapp W., Holubar K., Wick G. (Eds.) Elsevier/North Holland Biomedical Press. p. 215-224 (1978).

(11) Vollmer A., Bidwell OE., Bartlett A. in Bull Wld. Hlth. Org. 53, 55-56 (1976).

0168745

(12) Genentech Inc Veröffentlichung in Press J. Biol. Chem. (1984).

(13) Littlefield J. W., Science $\underline{145}$, 709 (1964).

(14) Fraker P.J., Speck J.C.Jr. Biochem. Biophys. Res. Com: $\underline{80}$, 849 (1978).

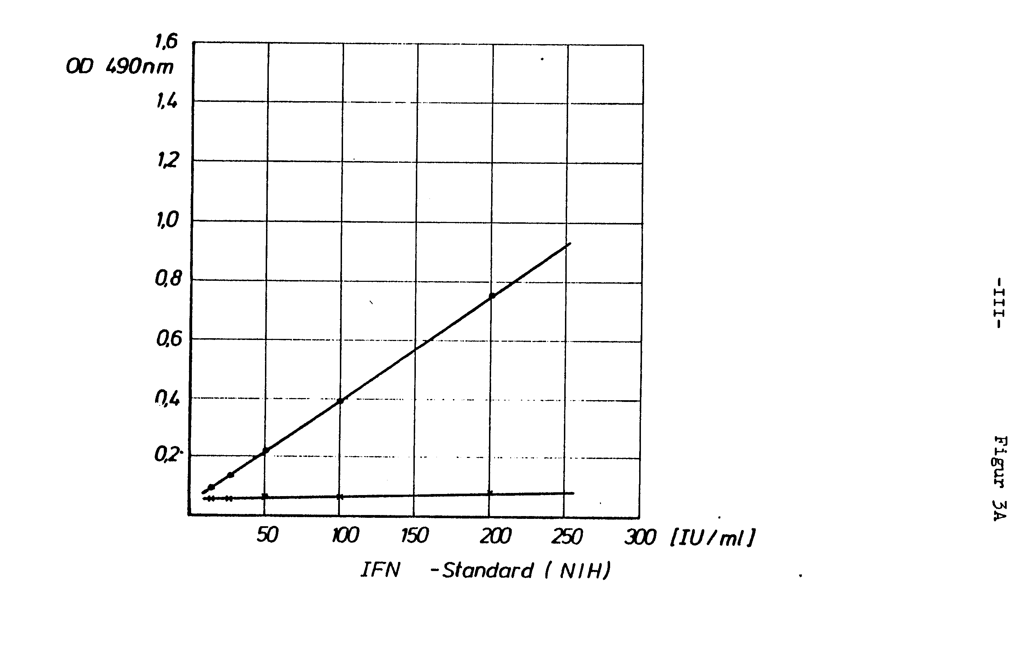

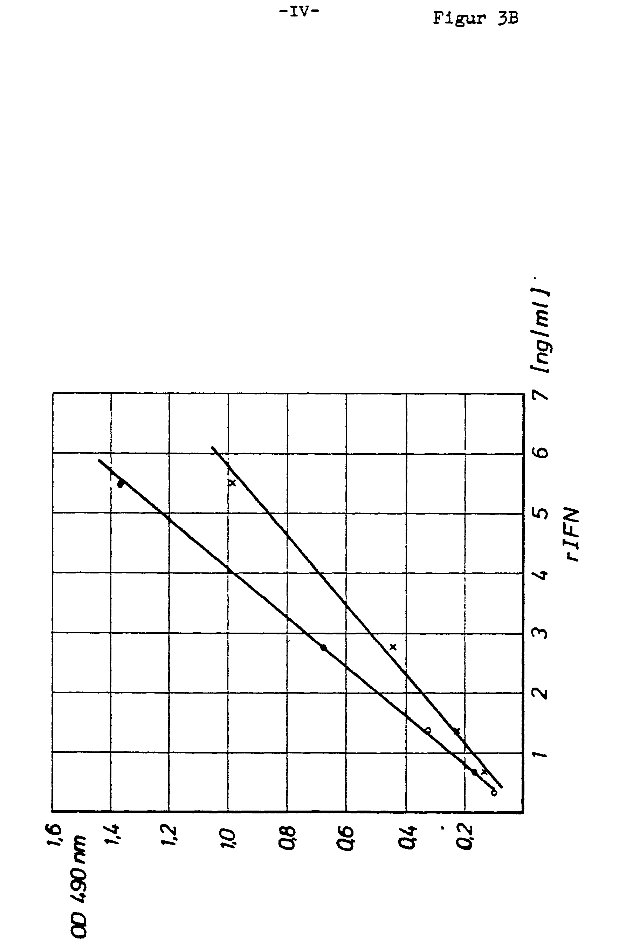

Legende zu den Figuren 1 bis 3

Figur 1: SDS-PAGE Analyse

Monoklonale Antikörper wurden aus Ascitesflüssigkeit gewonnen und nach der beschriebenen Methode gereinigt. Diese gereinigten Antikörper wurden mit Pepsin bei 37°C bis 2 Stunden verdaut. Die intakten Antikörper, so wie die isolierten F(ab')$_2$-Fragmente wurden zur Markierung verwendet (siehe Beispiel 2). Die Reinheit und das scheinbare Molekulargewicht der Antikörper und ihrer Fragmente wurde durch Elektrophorese in 8,5 % Polyacrylamidgelen in Gegenwart von SDS nach der Methode von Laemmli (7) bestimmt. Protein wurde durch Coomassie blau angefärbt. Die Position des intakten Antikörpers ist durch einen Pfeil gekennzeichnet.
1: C-15;   2: C-15 F(ab)$_2$;   3: GZ-4;   4: GZ-4 F(ab')$_2$;
5: GZ-25; 6: GZ-25 F(ab')$_2$;

Figur 2: Schematische Darstellung des Aufbaus des Immunoassays

Antikörper I und II können verschiedene Antikörper oder Formen eines einzigen Antikörpers sein.

Figur 3: ELISA für IFN-γ

Die Enzymaktivität des GZ-4-Peroxidasekonjugats mit dem Substrat OPD wurde bei einer Wellenlänge von 490 nm in einem Mikrotiterplattenlesegerät (Microelisa Autoreader MR 580, Dynateck, Denkendorf) gemessen. Die direkten Messwerte für 200 μl Lösung in Miktrotiterplattennäpfchen sind aufgetragen. Der Assay wurde wie beschrieben durchgeführt. Zur Beschichtung wurde Antikörper GZ-4 (o -- o) oder C-15 (x -- x) genommen. In A wurde natürliches IFN-γ (NIH Standard Gg 23-90/-530) in Internationalen Einheiten und in B rekombinantes IFN-γ (ohne Cys-Tyr-Cys-N-terminus) als [Proteinkonzentration] nachgewiesen.

## Patentansprüche

1. Hybridzellinien, dadurch gekennzeichnet, daß sie durch Zellfusion von Milzzellen einer mit rekombinantem humanen IFN-γ immunisierten Maus mit Myelomzellen erhalten werden, und die monoklonale Antikörper prodzieren, die mit natürlichem und/oder rekombinantem IFN-γ und deren Derivaten reagieren.

2. Hybridzellinie gemäß Anspruch 1, dadurch gekennzeichnet, daß als Milzzellen die von BALB/cMäusen und als Myelomzellen die der Linie P3-X-63Ag8-6-5-3 verwendet werden.

3. Hybridzellinie GZ-25 gemäß den Ansprüchen 1 und 2, dadurch gekennzeichnet, daß sie permanent als einzigen Antikörper den monoklonalen Antikörper GZ-25 produziert.

4. Hybridzellinie C-15 gemäß den Ansprüchen 1 und 2, dadurch gekennzeichnet, daß sie permanent als einzigen Antikörper den monoklonalen Antikörper C-15 produziert.

5. Hybridzellinie GZ-4 gemäß den Ansprüchen 1 und 2, dadurch gekennzeichnet, daß sie permanent als einzigen Antikörper den monoklonalen Antikörper GZ-4 produziert.

6. Monoklonale Antikörper, dadurch gekennzeichnet, daß sie mit natürlichem und/oder rekombinantem IFN-γ und deren Derivaten reagieren.

7. Monoklonaler Antikörper GZ-25 des Typs IgGl gemäß Anspruch 6, dadurch gekennzeichnet, daß
   a) sein Molekulargewicht > 150.000 Dalton beträgt,
   b) die schwere Kette γl, die leichte Kette kappa ist,
   c) er keine neutralisierende Wirkung auf die antivirale Aktivität von natürlichem und rekombinantem IFN-γ ausübt,

d) er eine bindende Wirkung auf rIFN-γ, welches nach der am N-terminalen Ende der Aminosäuresequenz die Sequenz $Cys^1$-$Tyr^2$-$Cys^3$- enthält, aufweist

e) er nur eine stark reduzierte bindende Wirkung auf rIFN-γ, welches nicht die Sequenz $Cys^1$-$Tyr^2$-$Cys^3$- enthält, aufweist und

f) er keine bindende Wirkung auf natürliches IFN-γ aufweist.


8. Monoklonaler Antikörper C-15 des Typs IgGl gemäß Anspruch 6, dadurch gekennzeichnet, daß

a) sein Molekulargewicht $\geqslant$ 150.000 Dalton beträgt,

b) die schwere Kette γl, die leichte Kette kappa ist,

c) er keine neutralisierende Wirkung auf die antivirale Aktivität von natürlichem und rekombinantem IFN-γ ausübt,

d) er eine bindende Wirkung auf rIFN-γ, unabhängig vom Vorhandensein der Sequenz $Cys^1$-$Tyr^2$-$Cys^3$-, aufweist,

e) er keine bindende Wirkung auf natürliches IFN-γ aufweist.


9. Monoklonaler Antikörper GZ-4 des Typs IgGl gemäß Anspruch 6, dadurch gekennzeichnet, daß

a) sein Molekulargewicht $\geqslant$ 150.000 Dalton beträgt,

b) die schwere Kette γl, die leichte Kette kappa ist,

c) er neutralisierende Wirkung auf die antivirale Aktivität von natürlichem und rekombinantem IFN-γ ausübt,

d) er eine bindende Wirkung auf rIFN-γ unabhängig vom Vorhandensein der Sequenz $Cys^1$-$Tyr^2$-$Cys^3$- aufweist,

e) er eine bindende Wirkung auf natürliches IFN-γ aufweist.


10. Verfahren zur Herstellung von neuen monoklonalen Antikörpern der Maus mit Anti-Humaninterferon-Spezifität gemäß Anspruch 6, dadurch gekennzeichnet, daß eine die Antikörper produzierende Hybridzellinie durch Zellfusion hergestellt und subkloniert wird und nach Wachstum der Zellen die monoklonalen Körper mit Anti-Humaninterferon-Spezifität isoliert werden.

11. Verfahren gemäß Anspruch 10, dadurch gekennzeichnet, daß zur Zellfusion Milzzellen von gegen IFN-γ immunisierten Mäusen verwendet werden.

12. Verfahren gemäß Anspruch 10, dadurch gekennzeichnet, daß als Myelomzellen ein Subklon der Zellinie P3-X-63Ag8-6-5-3 verwendet wird.

13. Verfahren gemäß Anspruch 11, dadurch gekennzeichnet, daß als Milzzellen Zellen von gegen Human-IFN-γ immunisierten BALB/c-Mäusen verwendet werden.

14. Verfahren gemäß den Ansprüchen 11 und 13, dadurch gekennzeichnet, daß rIFN-γ verwendet wird.

15. Verfahren zur Reinigung von rekombinantem oder natürlichem IFN-γ, dadurch gekennzeichnet, daß diese chromatographisch aufgrund der bindenden Wirkung der monoklonalen Antikörper gemäß den Ansprüchen 6 bis 9 auf diese Interferone über eine entsprechende Affinitätssäule erfolgt.

16. Verfahren zur Herstellung eines IgG-Antikörper-Affinitätsträgers, dadurch gekennzeichnet, daß ein gemäß Anspruch 10 hergestellter IgG-Antikörper kovalent an einen aktivierten Träger gebunden ist.

17. Verwendung der monoklonalen Antikörper gemäß den Ansprüchen 6 bis 9,

a) zum Nachweis von natürlichem oder rekombinantem IFN-γ, wobei sowohl die Interferon-Bindungen als auch die Interferon-neutralisierenden Eigenschaften der Antikörper eingesetzt werden,

b) zur diagnostischen Unterscheidung von natürlichem IFN-γ und von rIFN-γ durch unterschiedliche Bindung an die einzelnen Subtypen,

c) zur Reinigung von Immuninterferon, sowohl einzelner Subtypen desselben, als auch von Gemischen von γ-Interferonsub-
typen und deren Abtrennung von anderen Interferontypen.

18. Verwendung der monoklonalen Antikörper und geeigneter
Derivate davon gemäß den Ansprüchen 6 bis 9 zum Nachweis,
zur Diagnose oder zur Reinigung von IFN-γ, wobei jeder der
Antikörper und seine Derivate $F(ab')_2$ bzw. Fab für sich
allein, in beliebiger Kombination miteinander, in Kombination mit anderen monospezifischen Antikörpern miteinander,
in Kombination mit anderen monospezifischen Antikörpern oder
in Kombination mit polyklonalen Antikörpergemischen eingesetzt werden kann.

Figur 2

*Festphase*

I

IFN

II

Signal

Figur 3B